# EUROPEAN PATENT APPLICATION

(11) **EP 4 417 187 A1**
(43) Date of publication of application: **21.08.2024**
(21) Application number: 23305210.9
(22) Date of filing: 16.02.2023
(51) Int. Cl.: A61K 8/37, A61K 8/92, A61Q 1/06, A61Q 19/00, C07C 69/30

(54) **POLYOL ESTERS OF BRANCHED C18 FATTY ACIDS**

(71) Applicant: Oleon N.V., 9940 Ertvelde (BE)
(72) Inventor: VERHOEVEN, Luca, 9060 Zelzate (BE); DE SCHRIJVER, Bert, 9270 Kalken (BE); VAN HOLEN, Jurgen, 9400 Ninove (BE); STRUELENS, Pieter, 1755 Gooik (BE)
(74) Representative: Santarelli

(57) **Abstract**

The present invention relates to a mixture of polyol esters of branched C18 fatty acids, the process for preparing such a mixture and its use in various industrial fields, such as lubricants and cosmetics fields.

## Description

The present invention relates to a mixture of polyol esters of branched C18 fatty acids, the process for preparing such a mixture and its uses in various industrial fields, such as lubricants and cosmetics.

Commercially available branched fatty acid compositions such as "isostearic acid" (ISA), are obtained as a by-product of the catalytic and thermal dimerization of unsaturated linear fatty acid(s). Indeed, instead of oligomerizing, a portion of the fatty acid(s) rearranges to give branched monomeric fatty acids which can be isolated from the oligomerized fatty acids. This branched fatty acid composition commercially known as "isostearic acid" is a mixture of various linear and mainly branched, both mono and polybranched, saturated monocarboxylic fatty acids.

Esters derived from those "isostearic acid" are known and used in lubricant compositions and cosmetic products.

In lubricant compositions, some esters of branched fatty acids are used as base oil or additives.

In cosmetic products, some esters of branched fatty acids are used as emollients.

In particular, WO 2013/093411 describes the use of monoesters of formula I, R¹-COOR², wherein R¹ and R² are hydrocarbon chains; wherein at last 60% by weight of the molecules of formula I comprise a mono alkyl-branched R¹ and less than 25% by weight of the molecules of formula I comprise a poly alkyl-branched R¹, as emollients.

The Applicant surprisingly found that polyol esters obtained from a specific composition of branched fatty acids, present lower viscosities, better water resistance, better film forming and/or greater hardness, than corresponding esters obtained from commercial compositions of branched fatty acids with different features.

Those properties are particularly interesting for some lubricant compositions and cosmetic products.

Accordingly, the present invention relates to a mixture of polyol esters of branched C18 fatty acids, wherein said mixture is obtainable by esterification of a branched C18 fatty acid composition with a polyol, said composition comprising:
- at least 80% by weight of monobranched C18 fatty acids and polybranched C18 fatty acids; wherein the weight ratio monobranched C18 fatty acids / polybranched C18 fatty acids is greater than 1;
- at most 15% by weight of linear fatty acid(s);
- at most 10% by weight of cyclic compounds;
weight percentages being based on the weight of the composition.

This specific mixture of polyol esters of branched C18 fatty acids, presents lower viscosities than others mixtures of polyol esters of branched C18 fatty acids obtained from branched C18 fatty acid compositions having features different from the claimed ones. In particular, the kinematic viscosity at 40°C of the mixture of polyol esters according to the invention is at least 15% lower than the kinematic viscosity at 40°C of corresponding polyol esters obtained from branched C18 fatty acid compositions with features different from the claimed ones.

By "branched" fatty acid, it is intended to mean that the hydrocarbon chain of the fatty acid bears one or more alkyl side group(s), which is/are generally short.

By "short alkyl side group", it is intended to mean a group comprising less than 5 carbon atoms. More particularly, each short alkyl side group is linear and still more particularly, is chosen among the group constituted by methyl, ethyl and propyl. Preferably each short alkyl side group is a methyl and/or an ethyl, more preferably a methyl.

By "monobranched" fatty acid, it is intended to mean that the linear hydrocarbon chain of the fatty acid bears only one alkyl side group, which is generally short.

By "polybranched" fatty acid, it is intended to mean that the linear hydrocarbon chain of the fatty acid bears two or more alkyl side groups, which are generally short.

In the term "CX", X indicates the number of carbon atoms in the fatty acid, in other words, the number of carbon atoms in the hydrocarbon chain plus the optional alkyl side group(s). Consequently, "branched C18 fatty acids" designate all the branched fatty acids having 18 carbon atoms. In particular, these branched fatty acids are position isomers.

By "CX-CY fatty acids", it is then intended to mean that the number of carbon atoms in each fatty acid is comprised independently between X and Y.

In the present application, unless otherwise indicated, all ranges of values used are to be understood as being inclusive limits.

Preferably, the quantity of monobranched C18 fatty acids and polybranched fatty acids in the branched C18 fatty acid composition is of at least 82% by weight, more preferably of at least 84% by weight based on the weight of the composition.

Preferably, the quantity of monobranched C18 fatty acids and polybranched C18 fatty acids in the branched C18 fatty acid composition is of at most 98% by weight based on the weight of the composition.

The polyol is a linear or branched hydrocarbon chain comprising two or more hydroxyl groups.

The polyol preferably consists only of carbon, oxygen and hydrogen atoms.

Preferably, the polyol is saturated.

Suitable polyols for the present invention are, but not limited to trimethylolpropane, pentaerythritol, neopentyl glycol, glycerol, ethylene glycol, propylene glycol and 1,3-propanediol.

Preferably, the polyol is selected from the group consisting of neopentyl glycol, pentaerythritol and trimethylolpropane.

More preferably, the polyol is pentaerythritol or trimethylolpropane.

Preferably, in the mixture of polyol esters of branched C18 fatty acids according to the invention, the mean degree of esterification is higher than 1.

More preferably, in the mixture of polyol esters of branched C18 fatty acids according to the invention, all hydroxyl groups of the polyol are esterified.

The linear fatty acid(s) preferably comprise from 6 to 24 carbon atoms.

Preferably, the linear fatty acid(s) is/are saturated.

Preferably, there is no unsaturated fatty acid in the mixture of polyol esters according to the invention.

Preferably, there is no unsaturated compound in the mixture of polyol esters according to the invention.

Advantageously, in the mixture of polyol esters according to the invention, the quantity of linear fatty acid(s) is of at most 10% by weight based on the weight of the composition.

Preferably, the quantity of linear fatty acid(s) is of at most 8% by weight, more preferably of at most 7% by weight, even more preferably of at most 6% by weight based on the weight of the composition.

Preferably, the quantity of linear fatty acid(s) ranges from 1% to 10% by weight, more preferably from 1% to 8% by weight, even more preferably from 1% to 7% by weight, such as from 1% to 6% by weight based on the weight of the composition.

The cyclic compounds include but are not limited to alicyclic carboxylic acids or esters thereof, aromatic(s), alkylcyclopentanone(s), lactone(s) and mixture thereof.

Preferably, the cyclic compounds comprise from 14 to 22 carbon atoms, more preferably from 16 to 18 carbon atoms.

Advantageously, in the mixture of polyol esters according to the invention, the quantity of cyclic compounds is of at most 8% by weight based on the weight of the composition.

Preferably, the quantity of cyclic compounds is of at most 7%, more preferably of at most 6% by weight based on the weight of the composition.

Preferably, the quantity of cyclic compounds ranges from 1% to 8% by weight, more preferably from 1% to 7% by weight, even more preferably from 1% to 6% by weight, such as from 1% to 5% by weight, based on the total weight of the composition.

Advantageously, in the mixture of polyol esters according to the invention, the quantity of monobranched C18 fatty acids is of at least 45% by weight based on the weight of the composition.

Preferably, the quantity of monobranched C18 fatty acids is of at least 50% by weight based on the weight of the composition.

Preferably, the mixture according to the invention does not comprise a polyol ester of oligomerized fatty acid. More particularly, the branched C18 fatty acid composition does not comprise oligomerized fatty acid.

An oligomerized fatty acid is in particular a dimer, trimer or tetramer fatty acid.

In a preferred embodiment, the branched C18 fatty acid composition comprises:
- 80-98% by weight of monobranched C18 fatty acids and polybranched C18 fatty acids;
- 1-8% by weight of linear fatty acid(s);
- 1-8% by weight of cyclic compounds;

wherein the weight ratio monobranched C18 fatty acids / polybranched C18 fatty acids is greater than 1;
weight percentages being based on the weight of the composition.

In a particularly preferred embodiment, the branched C18 fatty acid composition comprises:
- 84-98% by weight of monobranched C18 fatty acids and polybranched C18 fatty acids; wherein the quantity of monobranched C18 fatty acids is of at least 50% by weight;
- 1-6% by weight of linear fatty acid(s);
- 1-6% by weight of cyclic compounds;

wherein the weight ratio monobranched C18 fatty acids / polybranched C18 fatty acids is greater than 1;
weight percentages being based on the weight of the composition.

The present invention also relates to a process for preparing a mixture of polyol esters of branched C18 fatty acids, comprising the esterification of a branched C18 fatty acid composition with a polyol;
wherein the branched C18 fatty acid composition comprises:
   - at least 80% by weight of monobranched and polybranched C18 fatty acids, with a weight ratio monobranched C18 fatty acids / polybranched C18 fatty acids greater than 1;
   - at most 15% by weight of linear fatty acid(s); and
   - at most 10% by weight of cyclic compounds;
weight percentages being based on the weight of the branched C18 fatty acid composition.

The branched C18 fatty acids, the polyol, the linear fatty acid(s) and the cyclic compounds are as described above, including preferential and advantageous positions.

The esterification reaction may be carried out according to any well-known conventional reaction conditions.

The esterification reaction is preferably conducted under stirring and heating at a temperature of at least 100°C, more preferably at least 120°C. In particular The esterification reaction may be carried out at a temperature comprised between 100 and 250°C, preferably between 120 and 240°C.

An esterification catalyst may be used, such as methanesulfonic acid.

Advantageously, the water is removed as it forms from the reaction mixture.

Preferably, the esterification reaction is carried out with at least one molar equivalent of branched C18 fatty acids.

More preferably, the molar ratio branched C18 fatty acids / polyol is of at least 1.

Preferably, the esterification reaction is carried out until the acid value is less than 20, preferably less than 15 mg KOH/g, the acid value being measured according to standard AOCS Cd 3D-63.

In the present application, unless otherwise indicated, all acid values are measured according to standard AOCS Cd 3D-63.

Preferably, the esterification reaction is carried out until the hydroxyl value is less than 100 preferably less than 25 mg KOH/g, the hydroxyl value being measured according to standard ASTM D1957.

In the present application, unless otherwise indicated, all hydroxyl values are measured according to standard ASTM D1957.

Advantageously, the process according to the invention, further comprise the preparation of the branched C18 fatty acid composition from a starting material comprising at least 80% by weight of linear monoethylenically unsaturated C18 fatty acid(s) based on the total weight of the starting material, said preparation comprising the steps of:
i) isomerizing the linear unsaturated C18 fatty acid(s) by heating in the presence of a zeolite catalyst having an orthorhombic framework with a one-dimensional straight channel of 10-membered-rings or with a two-dimensional channel system of 10-membered rings intersected by 8-membered rings;
ii) separating the monomeric fraction from the oligomeric fraction formed during step i);
iii) purifying the monomeric fraction to obtain the branched C18 fatty acid composition.

The linear unsaturated C18 fatty acids is composed of monoethylenically and polyethylenically unsaturated C18fatty acid(s).

Preferably, the linear unsaturated C18 fatty acids are oleic acid and/or elaidic acid, and linoleic acid and/or linolenic acid.

The linear monoethylenically unsaturated C18fatty acid(s) may be oleic acid and/or elaidic acid. Preferably, the linear monoethylenically unsaturated C18 fatty acid(s) is oleic acid.

The starting material is advantageously fatty acids of a renewable oil. A renewable oil is preferably a vegetable oil or an animal oil.

Some renewable oils comprise naturally at least 80% by weight of oleic acid based on the weight of the renewable oil. This fatty acid may be recovered from one of these oils by any known method in the art. Preferably, the starting material is fatty acids obtained from high oleic sunflower oil.

Some renewable oils that are mono and polyethylenically unsaturated, but comprise less than 80% by weight of oleic acid group based on the weight of the renewable oil, may be partially hydrogenated to optimize their content, prior to the recovering of corresponding fatty acids. Suitable renewable oils to partially hydrogenate are rapeseed oil, corn oil, soya bean oil, sunflower oil, safflower oil and tall oil.

Fatty acids obtained from any renewable oil may be fractionated to isolate oleic acid and obtain an adapted starting material.

Preferably, the starting material comprises no more than 95% by weight, more preferably no more than 90% by weight of linear monoethylenically unsaturated C18 fatty acid(s), based on the total weight of the starting material.

Preferably, the starting material further comprises at least 5% by weight of linear polyethylenically unsaturated C18 fatty acid(s).

Preferably, linear polyethylenically unsaturated C18 fatty acid(s) is/are linear diethylenically unsaturated C18 fatty acid(s), in particular linoleic acid.

Step i) is performed at a sufficient temperature to achieve an isomerization reaction. The isomerizing step may be conducted at a temperature ranging from 150°C to 300°C, preferably from 180°C to 260°C, more preferably from 230 to 250°C, and at a pressure ranging from 2.10⁵ Pa to 11.10⁵ Pa, preferably from 3.10⁵ Pa to 9.10⁵ Pa.

The isomerizing step may be conducted during 2 hours to 16 hours, preferably during 6 hours to 12 hours.

The isomerizing step may be performed in the presence of water, the water content ranging preferably from 0.1 to 5% by weight based on the total weight of the starting material.

The isomerizing step may be followed by an additional step of separation of the zeolite catalyst from the reaction product of step i), preferably by filtration.

Step ii) is preferably achieved by distillation, in particular by molecular distillation, at a temperature ranging from 200 to 300°C and at a pressure ranging from 1 to 4 mbar.

Step iii) can comprise hydrogenation, crystallization, fractionation and/or distillation.

Hydrogenation may be carried out by methods known in the art, for example by using palladium on carbon or supported nickel as a catalyst. Preferably, the temperature during hydrogenation ranges from 180 to 250°C and the pressure ranges from 16.10⁵ Pa to 26.10⁵ Pa.

Crystallization may be carried out using sulfate salts or urea (chlatration process), or at low temperatures, such as at 10°C or lower temperatures (cold crystallization), to separate the resulting solidified linear fatty acids from liquid branched fatty acids.

Distillation is preferably conducted at a temperature ranging from 200 to 300°C and at a pressure ranging from 1 to 4 mbar.

Preferably, step iii) comprises hydrogenation, crystallization, fractionation and distillation.

After step iii), a branched C18 fatty acid composition is obtained, comprising at least 80% by weight of branched C18 fatty acids, wherein the weight ratio monobranched C18 fatty acids/polybranched C18 fatty acids is greater than 1, less than 15 % by weight of linear fatty acid(s), and less than 10% by weight of cyclic compounds, weight percentages being given on the weight of the branched C18 fatty acid composition.

In a preferred embodiment, the process for preparing a mixture of polyol esters of branched C18 fatty acids, comprises:
- the preparation of a branched C18 fatty acid composition by:
   i) isomerizing the linear unsaturated C18 fatty acid(s) of a starting material comprising at least 80% by weight of linear monoethylenically unsaturated C18 fatty acid(s) based on the total weight of the starting material, by heating in the presence of a zeolite catalyst having an orthorhombic framework with a one-dimensional straight channel of 10-membered-rings or with a two-dimensional channel system of 10-membered rings intersected by 8-membered rings;
   ii) separating the monomeric fraction from the oligomeric fraction formed during step i);
   iii) purifying the monomeric fraction to obtain the branched C18 fatty acid composition;
- the esterification of the branched C18 fatty acid composition with a polyol.

The processes according to the invention allow the obtention of mixtures according to the invention. Thus the mixture of polyol esters according to the invention is obtainable by the process according to the invention, which is a process economically viable.

In the zeolite catalyst having an orthorhombic framework with a two-dimensional channel system of 10-membered rings intersected by 8-membered rings, preferably, both 10 and 8-member rings are elliptical in shape with dimensions of respectively 4.2×5.4 Angstroms and 3.5×4.8 Angstroms.

The zeolite catalyst is preferably a zeolite catalyst of the MTT, TON or FER framework type. In particular, the zeolite catalyst is ZSM-22, ZSM-23 or ferrierite (FER).

Preferably, the zeolite catalyst is calcined, prior to its use in the isomerizing step.

More particularly, the zeolite catalyst is H-ZSM-22, H-ZSM-23 or H-FER.

Preferably, the quantity of zeolite catalyst is of at least 0.1% by weight, more preferably of at least 0.5% by weight, even more preferably of at least 1% by weight based on the weight of the starting material.

Preferably, the quantity of zeolite catalyst is of at most 10% by weight, more preferably of at most 7% by weight, even more preferably of at most 5% by weight based on the weight of the starting material.

The present invention also concerns the use of the mixture of polyol esters according to the invention, in a lubricant composition.

As illustrated in Example 3, mixtures of polyol esters of branched C18 fatty acids according to the invention present lower viscosities than corresponding polyol esters of branched C18 fatty acids having different features.

Thus, mixtures of polyol esters according to the invention are preferred in applications which require low viscosity lubricants.

Therefore, the invention also relates to a lubricant composition comprising the mixture of polyol esters according to the invention and a base oil.

Preferably, the base oil content is of at least 50% by weight, more preferably at least 75% by weight based on the weight of the lubricant composition.

Preferably, the quantity of mixture of polyol esters according to the invention is of at least 2% by weight, more preferably at least 5% by weight based on the weight of the lubricant composition.

Preferably, the quantity of mixture of polyol esters according to the invention is of at most 50% by weight, more preferably at most 45% by weight based on the weight of the lubricant composition.

The base oil may comprise one or more oils chosen among mineral oils, renewable oils and/or synthetic oils. Preferably, the base oil is chosen from the group consisting of mineral oils and/or synthetic oils.

Mineral oils are oils obtained from petroleum refining. They consist essentially of carbon and hydrogen atoms, such as paraffinic oils, hydrorefined oils, hydrocracked oils and hydro-isomerized oils.

Mineral oils are categorized into three groups:
- group I oils: these oils have a saturated hydrocarbon content less than 90% by weight, an aromatic hydrocarbon content higher than 1.7% by weight, a sulfur content higher than 0.03% by weight, and a viscosity index between 80 and 120;
- group II oils: these oils have a saturated hydrocarbon content higher than 90% by weight, an aromatic hydrocarbon content less than 1.7% by weight, a sulfur content less than 0.03% by weight, and a viscosity index between 80 and 120;
- group III oils: these oils have a saturated hydrocarbon content higher than 90% by weight, an aromatic hydrocarbon content less than 1.7% by weight, a sulfur content less than 0.03% by weight, and a viscosity index higher than 120;
weight percentages being based on the weight of the oil.

Synthetic oils are obtained by chemical reaction between molecules of petrochemical origin and/or of renewable origin, with the exception of the usual chemical reactions used to obtain mineral oils (such as hydrorefining, hydrocracking, hydrotreating). hydroisomerization, etc.). Examples of synthetic oils, are esters, polyalkylene glycols (PAG) and polyalphaolefins (PAO). Preferably, synthetic oil is a polyalkylene glycol (PAG) a polyalphaolefin (PAO) or a mixture thereof.

Lubricant compositions of the invention find advantageously applications in the industrial sector and the automotive sector.

Examples of lubricant compositions of industrial sector are textile fiber oils, industrial transmission oils, compressor oils, turbine oils, gear oils and hydraulic oils.

Examples of lubricant compositions of automotive sector are hydraulic oils, transmission fluids, cooling fluids, engine oils, oils for axles, gearbox fluids, brake fluids, shock-absorber oils and damper oils.

In the present patent application, the terms "oil" and "fluid" are used interchangeably in the designation of the applications/uses of the compositions according to the invention.

Preferably, the mixture of polyol esters according to the invention can be used for the preparation of a lubricant composition for the automotive sector and/or for the industrial sector.

The present invention also concerns the use of the mixture of polyol esters according to the invention, in a cosmetic product.

In cosmetics, in particular in personal care products, mixtures of polyol esters according to the invention, are also useful as they exhibit good film forming, water resistance and hardness properties, as illustrated in Example 4.

Those properties are particularly of interest for cosmetic products. Indeed, the film forming property prevents dehydration of the skin. The water resistance property allows the cosmetic product to stay longer on skin by resisting to washing and/or sweating. The hardness property allows the formation of solid cosmetic products.

The invention also relates to a cosmetic product, in particular a personal care product, comprising a mixture of polyol esters according to the invention and an active ingredient and/or a preservative.

Preferably, the active ingredient is an UV filter, an anti-aging agent and/or a hydrating agent.

More particularly, the active ingredient is chosen among the group consisting of allantoin, tocopherol, ascorbic acid, ascorbyl palmitate, vitamin D, polyphenols, flavonoids, and mixtures thereof.

More particularly, the preservative is chosen among the group consisting of phenoxyethanol, glycol ethers, benzoic acid, sorbic acid, salicylic acid, glycols, parabens, thiazolinones, ethylhexylglycerin, aldehydes, and mixtures thereof.

Preferably, the personal care product is a lipstick, a lip gloss, a hydrating cream, a sun cream or a mascara.

The invention is further described in the following examples. It will be appreciated that the invention as claimed is not intended to be limited in any way by these examples.

### Example 1: Process for preparing mixtures of polyol esters of branched C18 fatty acids according to the invention

### 1.1 Preparation of branched C18 fatty acid compositions ISA1-ISA3

### 1.1.1 Isomerization of linear unsaturated fatty acids using zeolite catalyst

Three compositions of branched C18 fatty acids ISA1-ISA3 were prepared using different zeolite catalysts, respectively H-ZSM22, H-ZSM23 and ferrierite.

For each composition, 1200 g of fatty acids obtained from high oleic sunflower oil (comprising 83 wt% of oleic acid and 7.3 wt% of linoleic acid) and 30 g of a zeolite catalyst were placed together in an autoclave. Air was flushed out of the autoclave with nitrogen. While stirring, the mixture was heated to 240°C. This reaction temperature was held for 7 hours, the pressure had built up to 11.10⁵ Pa.

The reaction mixture was then cooled down to 80°C, while removing gaseous components by flushing with nitrogen.

The zeolite catalyst was subsequently removed from the reaction product by vacuum filtration.

### 1.1.2 Recovering of the monomeric fraction

The monomeric fraction, amounting to more than 90 wt%, was separated from the oligomeric fraction by distillation up to 260°C under 2 mbar.

### 1.1.3 Purification of the monomeric fraction

A hydrogenation step was conducted on the monomeric fraction with 0.20% of palladium on carbon catalyst. The product was hydrogenated at 200°C until the hydrogen consumption stopped.

Next, the product was further purified by crystallization in order to isolate the branched fatty acids. For doing this, an aqueous solution containing 1.2 wt% of sodium decyl sulfate and magnesium sulfate was added to the monomeric fraction and the mixture was cooled down to 10°C. The aqueous phase, together with crystals of mainly linear fatty acids, was removed from the branched fatty acids by centrifugation. The composition of branched fatty acids were washed three times with water.

Then, an identical aqueous solution was added to the branched fatty acids and the mixture was cooled down to 4°C. Like previously, the aqueous solution was then removed and the new composition of branched C18 fatty acids were washed three times with water.

In a final purification step, low molecular weight compounds were stripped of by means of fractionated distillation.

### 1.1.4 Analysis of the branched C18 fatty acid compositions

To characterize the branched C18 fatty acid compositions obtained after the purification step, the carboxylic acids of latter were esterified with methanol. A sample of each composition was then analyzed by gas chromatography according to standard ISO 12966-1:2014

The content of each composition of branched C16-C18 fatty acids obtained is described in Table 1 below.

**Table 1: Branched C18 fatty acid compositions suitable for the invention**

| | | ISA 1 | ISA 2 | ISA 3 |
|---|---|---|---|---|
| Zeolite catalyst | | H-ZSM-22 | H-ZSM-23 | Ferrierite |
| | | | | |
| Branched C18 fatty acids | | 91.4 | 92.6 | 94.6 |
| | Monobranched C18 fatty acids | 59.3 | 59.0 | 87.1 |
| | Polybranched C18 fatty acids | 32.1 | 33.6 | 7.5 |
| Cyclic compounds | | 3.7 | 2.7 | 2.1 |
| Linear fatty acids | | 4.7 | 4.3 | 3.2 |
| | Linear C14 fatty acid | 0.0 | 0.1 | 0.0 |
| | Linear C16 fatty acid | 2.4 | 2.3 | 1.2 |
| | Linear C18 fatty acid | 1.6 | 1.6 | 1.3 |
| | Linear C20 fatty acid | 0.5 | 0.2 | 0.6 |
| | Linear C22 fatty acid | 0.2 | 0.1 | 0.1 |
| Ratio monobranched / polybranched | | 1.84 | 1.75 | 11.6 |

### 1.2 Preparation of mixtures of polyol esters according to the invention

The branched C18 fatty acid compositions ISA 1 and ISA 3 were esterified with respectively trimethylolpropane (trimethylolpropane flakes from Perstorp) to form triesters, and pentaerythritol (Voxtar M40 from Perstorp) to form tetraesters.

The esterification reaction conducted until the acid value was constant and the hydroxyl value was below 5 mgKOH/g; the acid value being measured according to the standard AOCS Cd 3D-63, and the hydroxyl value being measured according to the standard D1957.

After filtration using dicalite (0.5 wt%), excess of isostearic acid was removed by distillation.

### Example 2: Comparative mixtures of polyol esters of branched C18 fatty acids

### 2.1 Preparation of comparative mixtures of polyol esters of a branched C18 fatty acid composition

Esterification of two branched C18 fatty acid compositions C1 (Radiacid 5909 from Oleon) and C2 (Radiacid 0909 from Oleon) not suitable for the invention were conducted according to the method described in Example 1.2 using trimethylolpropane and pentaerythritol as polyols.

**Table 2: Comparative isostearic acid compositions C1 and C2**

| | | C1 | C2 |
|---|---|---|---|
| Branched C18 fatty acids | | 83.7 | 70.5 |
| | Monobranched C18 fatty acids | 31.8 | 38.4 |
| | Polybranched C18 fatty acids | 51.9 | 32.1 |
| Cyclic fatty acids | | 6.9 | 19.5 |
| Linear fatty acids | | 8.9 | 7.9 |
| | Linear C14 fatty acid | 0.2 | 0.1 |
| | Linear C16 fatty acid | 6.0 | 4.2 |
| | Linear C18 fatty acid | 2.3 | 2.3 |
| | Linear C20 fatty acid | 0.3 | 0.8 |
| | Linear C22 fatty acid | 0.1 | 0.3 |
| Ratio monobranched / polybranched | | 0.61 | 1.19 |

### Example 3: Viscosities of mixtures of polyol esters

### 3.1 Determination of kinematic viscosities

Kinematic viscosities at 40°C were determined according to the standard ASTM D445. Results obtained for each mixture of polyol esters of a branched C18 fatty acid composition are gathered in Table 3 below:

**Table 3: Kinematic viscosities**

| | Kinematic viscosity at 40°C (mm²/s) |
|---|---|
| Trimethylolpropane triisostearate | |
| from ISA 1 | 81.4 |
| from ISA 3 | 79.1 |
| from C1 | 99.7 |

| Pentaerythritol tetraisostearate | |
|---|---|
| from ISA 3 | 110.1 |
| from C1 | 153.6 |

As can be seen, mixtures of polyol esters comprising at least 80 wt% of polyol esters of branched C18 fatty acids, with a higher content of monobranched than polybranched, have a lower kinematic viscosity.

### 3.2 Determination of dynamic viscosities

Dynamic viscosities were determined according to the standard ISO 2884-2. Results obtained for each mixture of polyol esters of branched C18 fatty acids are gathered in Table 4 below:

**Table 4: Dynamic viscosities**

| | Dynamic viscosity at 25°C (mPa.s) |
|---|---|
| Trimethylolpropane triisostearate | |
| from ISA 1 | 156.6 |
| from C2 | 202.0 |

| Pentaerythritol tetraisostearate | |
|---|---|
| from ISA 1 | 237.1 |
| from C2 | 310.0 |

As can be seen, mixtures of polyol esters comprising at least 80 wt% of polyol esters of branched C18 fatty acids, with a higher content of monobranched than polybranched and a low content of cyclic compounds (2.7wt% for ISA1 vs 19.5 wt% for C2), have a lower dynamic viscosity.

### Example 4: Cosmetic products comprising polyol isostearic acid esters

### 4.1 Lipsticks

Chemicals and their quantities are described in Table 5 below.

To prepare a lipstick, phase A was prepared by introducing all the ingredients into the main beaker, and heated at 80°C until a clear solution was obtained.

Phase B was prepared by mixing the pigments and the pearling agent with the emollient in a separate beaker under stirring until a homogeneous paste was obtained.

Phase B was added to phase A and the heating was stopped.

Once the temperature decreased to 50°C, ingredients of phase C were added to the mixture of phases A + B.

The resulting mixture was poured into a specific mold, which was then placed at 4°C for 15 minutes.

Once at room temperature, the lipstick was unmoulded.

**Table 5: Formulation of lipsticks L1 and L2 according to the invention**

| | INCI names | Function | L1 (%w/w) | L2 (%w/w) |
|---|---|---|---|---|
| A | Candelila wax | consistency factor | 12.0 | 12.0 |
| | Rice wax | consistency factor | 7.0 | 7.0 |
| | Myristyl myristate | consistency factor | 5.0 | 5.0 |
| | Butyrospermum parkii butter | skin conditioning | 2.0 | 2.0 |
| | Caprylic/capric triglycerides | emollient | 8.0 | 8.0 |
| | Glyceryl triethylhexanoate | emollient | 10.0 | 10.0 |
| | Mixture of poyol esters according to the invention: trimethylolpropane triisostearate from ISA 1 | emollient | 10.0 | - |
| | Mixture of poyol esters according to the invention: pentaerythritol tetraisostearate from ISA 1 | emollient | - | 10.0 |
| | Polyglyceryl-3-diisostearate | stick stabilizer | 10.0 | 10.0 |
| B | Isoamyl laurate | dispersing agent | 20.0 | 20.0 |
| | Synthetic Wax (And) Red 7 Lake (And) Isopropyl Titanium Triisostearate | pigment | 6.4 | 6.4 |
| | Red 21 Lake (and) Isononyl Isononanoate (an d) Isopropyl Myristate (and) Stearalkonium Hecto rite (and) Isopropyl Titanium Triisostearate (an d) Propylene Carbonate (and) Polyhydroxystearic A cid) | pigment | 3.6 | 3.6 |
| | (Titanium Dioxide (and) Polyglyceryl-4 Isostearate (and) Cetyl P EG/PPG-10/1 Dimethicone (and) Hexyl Laurate (and) Isopropyl Titanium Triisostearate) | pigment | 1.8 | 1.8 |
| | Mica Bronze | perling agent | 1.8 | 1.8 |
| C | Tocopherol acetate | antioxidant | 1.0 | 1.0 |
| | Flavour oil | fragrance | 0.4 | 0.4 |
| | Ethylhexyl methoxycinnamate | UV absorber | 1.0 | 1.0 |

To compare the efficiency of lipsticks according to the invention (L1), three comparative lipsticks were prepared:
- CL1 is a lipstick wherein the mixture of poyol esters according to the invention was replaced by a natural film-forming and water resistant polymer (Polyester-7 (and) Neopentyl Glycol Diheptanoate, Lexfilm SUN from Inolex);
- CL2 is a lipstick control comprising no mixture of polyol esters of branched C18 fatty acids, nor polymer, but more (20 wt%) of glyceryl triethylhexanoate;
- CL3 is a lipstick prepared using a comparative mixture of polyol esters of branched C18 fatty acids (Trimethylolpropane triisostearate from C2) instead of a mixture of polyol esters according to the invention; and
- CL4 is a lipstick prepared using a comparative mixture of polyol esters of branched C18 fatty acids (Pentaerythritol tetraisostearate from C2) instead of a mixture of polyol esters according to the invention.

### 4.2 Film forming

To evaluate the film forming property of the different lipsticks prepared in Example 4.1, the evaporation of water from a cup covered by a kraft paper where a film of lipstick was applied on, was measured.

The covered cup was kept in oven at 40°C for 48 hours.

The water content was measured and the difference of content before and after the 2 days in oven is indicated in percentage of loss in Table 6 below.

**Table 6: Film forming property**

| | Water loss (%) |
|---|---|
| L1: trimethylolpropane triisostearate from ISA 1 | 11.89 |
| CL3: trimethylolpropane triisostearate from C2 | 24.50 |
| L2: pentaerythritol tetraisostearate from ISA 1 | 12.02 |
| CL4: pentaerythritol tetraisostearate from C2 | 16.01 |
| CL1: polyester-7 (and) Neopentyl Glycol Diheptanoate | 16.62 |
| CL2: control | 27.95 |

It can be observed that lipsticks according to the invention L1 and L2, present the lowest water lost, respectively 11.89% and 12.02%. It can be concluded that those lipsticks comprising a mixture of polyol esters of branched C18 fatty acids exhibit a better film forming property than the other lipsticks, even better than the lipstick obtained using a polymer known to form a film (CL1 with a water loss of 16.62%).

### 4.3 Water resistance

To evaluate the water resistance property of lipsticks prepared in Example 4.1, the solubilisation of lipstick into water was measured.

Each lipstick was applied on a sheet of white cotton cloth pre-weighed (W0), which was then immersed in water in beaker stirred for 30 minutes.

Each sheet of cotton cloth was then kept at 80°C for 24 hours.

The sheet of cotton clothes were then weighted (W24). The percentage of lipstick remaining on the cloth is given in Table 7 below.

**Table 7: Water resistance property**

| | Lipstick remaining on cotton cloth (%) |
|---|---|
| L1: trimethylolpropane triisostearate from ISA 1 | 96.67 |
| CL3: trimethylolpropane triisostearate from C2 | 61.36 |
| L2: pentaerythritol tetraisostearate from ISA 1 | 86.67 |
| CL4: pentaerythritol tetraisostearate from C2 | 73.91 |
| CL1: polyester-7 (and) Neopentyl Glycol Diheptanoate | 76.92 |
| CL2: control | 44.12 |

It can be seen that an higher percentage of lipsticks according to the invention L1 and L2, remain on the cotton cloth, meaning that these lipsticks exhibit a better water resistance property than the other lipsticks.

### 4.4 Hardness

To measure the hardness of lipsticks, needle probe penetration tests were performed. The method for testing lipstick hardness is adapted from the standard ASTM D1321-10 using a 2 mm Needle Probe (TA39). Before the test, each lipstick was centred under the needle probe to facilitate their penetration. Measurements were performed in the following conditions:
- Test Type: Compression
- Pre-load Test Speed: 1.0 mm/s
- Test Speed: 10mm/s
- Post-Test Speed: 10.0 mm/s
- Target Value: 5 mm
- Trigger Force: 5 g (0.049N).

The measurement of hardness was repeated three times for each lipstick. The resulting average values of hardness are gathered in Table 8 below.

**Table 8: Hardness**

| | Average hardness (N) of lipsticks |
|---|---|
| L1: trimethylolpropane triisostearate from ISA 1 | 1.437 |
| CL3: trimethylolpropane triisostearate from C2 | 0.671 |
| L2: pentaerythritol tetraisostearate from ISA 1 | 1.235 |
| CL4: pentaerythritol tetraisostearate from C2 | 0.731 |
| CL1: polyester-7 (and) neopentyl glycol diheptanoate | 0.749 |
| CL2: control | 0.563 |

Lipsticks according to the invention present greater hardness than other lipsticks. By comparing the hardness of L1 (1.437) with the one of CL3 (0.671), and those of L2 (1.235) with CL4 (0.731), it can be observed an increase of at least 30% in the hardness. The use of a mixture of polyol esters of branched C18 fatty acids in a cosmetic product allows to improve the hardness of the product.

## Claims

1. Mixture of polyol esters of branched C18 fatty acids, wherein said mixture is obtainable by esterification of a branched C18 fatty acid composition with a polyol, said composition comprising:
- at least 80% by weight of monobranched C18 fatty acids and polybranched C18 fatty acids; wherein the weight ratio monobranched C18 fatty acids / polybranched C18 fatty acids is greater than 1;
- at most 15% by weight of linear fatty acid(s);
- at most 10% by weight of cyclic compounds;
weight percentages being based on the weight of the composition.

2. Mixture of polyol esters according to claim 1, wherein the quantity of linear fatty acid(s) is of at most 10% by weight based on the weight of the composition.

3. Mixture of polyol esters according to claim 1 or 2, wherein the quantity of cyclic compounds is of at most 8% by weight based on the weight of the composition.

4. Mixture of polyol esters according to any of claims 1 to 3, wherein the quantity of monobranched C18 fatty acids is of at least 45% by weight based on the weight of the composition.

5. Process for preparing a mixture of polyol esters of branched C18 fatty acids, comprising the esterification of a branched C18 fatty acid composition with a polyol; wherein the branched C18 fatty acid composition comprises:
- at least 80% by weight of monobranched and polybranched C18 fatty acids, with a weight ratio monobranched C18 fatty acids / polybranched C18 fatty acids greater than 1;
- at most 15% by weight of linear fatty acid(s); and
- at most 10% by weight of cyclic compounds;
weight percentages being based on the weight of the branched C18 fatty acid composition.

6. Process according to claim 5, further comprising the preparation of the branched C18 fatty acid composition from a starting material comprising at least 80% by weight of linear monoethylenically unsaturated C18 fatty acid(s) based on the total weight of the starting material, said preparation comprising the steps of:
i) isomerizing the linear unsaturated C18 fatty acid(s) by heating in the presence of a zeolite catalyst having an orthorhombic framework with a one-dimensional straight channel of 10-membered-rings or with a two-dimensional channel system of 10-membered rings intersected by 8-membered rings;
ii) separating the monomeric fraction from the oligomeric fraction formed during step i);
iii) purifying the monomeric fraction to obtain the branched C18 fatty acid composition.

7. Use of the mixture of polyol esters of any of claims 1 to 4, in a lubricant composition.

8. Use of the mixture of polyol esters of any of claims 1 to 4, in a cosmetic product.
